# FASCICULE DE BREVET EUROPEEN

(11) **EP 2 029 202 B1**
(45) Date de publication et mention de la délivrance du brevet: **25.12.2013**
(21) Numéro de dépôt: 07788884.0
(22) Date de dépôt: 13.06.2007
(51) Int. Cl.: A61M 5/32

(54) **DISPOSITIF SECURISE D'INJECTION D'UN PRODUIT MEDICAMENTEUX TEL QU'UN PRODUIT LYOPHILISE**
SICHERHEITSVORRICHTUNG ZUR INJEKTION EINES MEDIZINPRODUKTS, Z.B. EINES LYOPHILISERTEN PRODUKTS
SAFETY DEVICE FOR INJECTING A MEDICINAL PRODUCT, SUCH AS A LYOPHILIZED PRODUCT

(30) Priorité: 16.06.2006 US 814071 P
(43) Date de publication de la demande: 04.03.2009
(73) Titulaire: Brunel, Marc, 31000 Toulouse (FR)
(72) Inventeur: Brunel, Marc, 31000 Toulouse (FR)
(74) Mandataire: Hartmann, Jean-Luc
(86) Numéro de dépôt international: PCT/FR2007/000979
(87) Numéro de publication internationale: WO 2007/144507

(56) Documents cités:
- EP-A1- 0 467 173
- WO-A-93/00949
- WO-A-02/072182
- FR-A1- 2 861 310

## Description

L'invention concerne un dispositif sécurisé d'injection d'un produit médicamenteux tel qu'un produit lyophilisé.

Pour des raisons évidentes de sécurité, les dispositifs actuels d'injection à usage unique sont couramment équipés d'un dispositif de protection après usage de l'aiguille d'injection.

De façon usuelle, de tels dispositifs d'injection comprennent un corps de seringue comportant un récipient portant une aiguille d'injection et doté d'un piston actionné par une tige de piston dotée d'un bouton poussoir, et un dispositif de protection comportant :
- un étui de protection adapté pour que le dit étui de protection et le corps de seringue soient aptes à coulisser relativement l'un par rapport à l'autre entre une position d'injection dans laquelle l'aiguille d'injection s'étend au moins partiellement dans le prolongement de l'étui de protection et une position de protection après usage dans laquelle l'aiguille d'injection est entièrement logée dans l'étui de protection,
- et au moins un organe de verrouillage apte à présenter un état verrouillé de maintien du corps de seringue et de l'étui de protection dans leur position d'injection, et pour commuter vers un état déverrouillé apte à autoriser, en fin de course du piston à l'intérieur du récipient, le coulissement relatif du corps de seringue et de l'étui de protection vers leur position de protection après usage.

Il est, en outre, à noter que ce principe s'applique tant aux dispositifs dotés d'une aiguille d'injection scellée sur le récipient, qu'aux dispositifs d'injection comportant une aiguille d'injection rapportée et solidarisée de façon démontable par tout système d'assemblage connu en soi (cône Luër... ).

La conception même de ces dispositifs d'injection (déclenchement de la protection en fin de course du piston) leur interdit toutefois d'être utilisés notamment pour l'injection de médicaments requérant une reconstitution (solvant - produit lyophilisé) avant injection, et nécessitant, à cet effet :
- une première vidange destinée à injecter le solvant renfermé dans le dispositif d'injection dans un flacon contenant le produit lyophilisé,
- une aspiration du produit reconstitué en vue du remplissage du dispositif d'injection,
- et l'injection proprement dite du produit médicamenteux.

En effet, leur conception conduirait au déclenchement de la protection après la première vidange interdisant les deux étapes ultérieures.

En vue de pallier cette lacune, une solution notamment décrite dans les demandes de brevet FR 2861310 et WO 02/072182 a consisté à doter les dispositifs d'injection d'un « organe inhibiteur » adapté :
- du fait de sa seule présence, ou dans une première position de ce dernier, pour raccourcir la course du piston lors de la première vidange, afin d'éviter le déclenchement de la protection en fin de course,
- après son retrait, ou dans une seconde position de ce dernier, pour autoriser, lors de l'injection proprement dite, une course normale du piston provoquant le déclenchement de la protection en fin de course.

Une telle solution présente l'avantage de requérir seulement l'adjonction d'un simple « organe inhibiteur » en vue de parvenir au résultat escompté.

Par contre, il s'est avéré q'une telle solution qui nécessite une action spécifique s'écartant des gestes naturels utilisés pour l'injection de produits reconstitués, a été mal accueillie par le personnel médical, de sorte que les dispositifs conçus selon ce principe connaissent en fait un très faible succès commercial.

La présente invention vise à pallier cet inconvénient et a pour principal objectif de fournir un dispositif d'injection sécurisé permettant l'injection de produits à reconstituer selon une méthodologie strictement identique à celle mise en oeuvre lors de l'utilisation de dispositifs d'injection non sécurisés.

Un autre objectif de l'invention est de fournir un dispositif d'injection sécurisé entraînant un surcoût très faible par rapport aux dispositifs d'injection actuels non sécurisés.

A cet effet, l'invention vise un dispositif d'injection d'un produit médicamenteux tel qu'un produit lyophilisé présentant la spécificité de comporter un dispositif de protection comprenant :
- un épaulement ménagé en position intermédiaire de la longueur de la tige de piston,
- un élément de verrouillage solidarisé sur l'un des éléments, corps de seringue ou étui de protection, comportant :
   - des organes de blocage des organes de verrouillage, aptes à être déplacés entre une position initiale naturelle, dite de désactivation, de blocage de chaque organe de verrouillage dans son état verrouillé, et une position, dite d'activation, d'autorisation d'une commutation de chaque organe de verrouillage vers son état déverrouillé,
   - et des organes d'actionnement reliés aux organes de blocage, positionnés sur le trajet de l'épaulement intermédiaire de la tige de piston, et adaptés pour subir un changement d'état entraînant un déplacement des dits organes de blocage de leur position de désactivation vers leur position d'activation, lors d'une sollicitation exercée sur les dits organes d'actionnement par l'épaulement intermédiaire, lors d'un déplacement de la tige de piston résultant d'une traction exercée sue le bouton poussoir.

L'invention requiert donc principalement, afin de remplir l'objectif visé, d'équiper les dispositifs d'injection sécurisés classiques d'un élément de verrouillage, et de solidariser ce dernier, par exemple par encliquetage, sur un des éléments, corps de seringue ou étui de protection, de ces dispositifs d'injection. Par conséquent, le surcoût généré par la solution selon l'invention s'avère très faible.

De plus, la transition des organes de blocage entre leur position naturelle de désactivation des organes de verrouillage et leur position d'activation de ces organes de verrouillage, s'opère automatiquement, sans intervention humaine, lors de l'aspiration du produit reconstitué pour remplir le dispositif d'injection.

Par conséquent, la méthodologie mise en oeuvre en vue de l'injection de produits à reconstituer s'avère strictement identique à celle mise en oeuvre lors de l'utilisation de dispositifs d'injection non sécurisés.

Selon une première variante avantageuse de réalisation de l'invention :
- l'élément de verrouillage est solidarisé sur un des éléments, corps de seringue ou étui de protection, avec une faculté de débattement selon une direction parallèle à l'axe longitudinal du dispositif de protection, entre deux positions définissant les positions respectives de désactivation et d'activation des organes de blocage des organes de verrouillage,
- les organes d'actionnement comportent au moins un levier articulé sur l'élément de verrouillage autour d'un axe de pivotement et comportant, s'étendant de part et d'autre du dit axe de pivotement :
   - une zone de contact située sur le trajet de l'épaulement intermédiaire de la tige de piston,
   - et au moins une pièce d'arc-boutement apte à engendrer un débattement de l'élément de verrouillage entraînant un déplacement des organes de blocage de leur position de désactivation vers leur position d'activation, lors d'un pivotement du levier provoqué par le déplacement de la tige de piston résultant d'une traction exercée sue le bouton poussoir.

Selon une seconde variante avantageuse de réalisation de l'invention :
- l'élément de verrouillage est solidarisé en position fixe sur un des éléments, corps de seringue ou étui de protection,
- les organes d'actionnement comportent au moins une pièce de verrouillage solidaire de l'élément de verrouillage, comportant d'un seul tenant
   - une zone de contact située sur le trajet de l'épaulement intermédiaire de la tige de piston,
   - au moins un organe de blocage d'un organe de verrouillage,
   - et une zone déformable de liaison entre la zone de contact et chaque organe de blocage, apte à se déformer et à entraîner un déplacement de chaque organe de blocage de sa position de désactivation vers sa position d'activation, lors d'une sollicitation exercée sur la zone de contact par l'épaulement intermédiaire, lors d'un déplacement de la tige de piston résultant d'une traction exercée sue le bouton poussoir.

Par ailleurs, selon l'invention, l'élément de verrouillage est avantageusement solidarisé sur le corps de seringue.

De plus, en vue de parfaire le verrouillage des doigts de verrouillage, les organes de blocage de l'élément de verrouillage et les dits organes de verrouillage comprennent avantageusement des organes d'assemblage amovible complémentaires, du type cran/ ergot.

Par ailleurs, lorsque les organes de verrouillage, de maintien du corps de seringue et de l'étui de protection dans leur position d'injection, consistent de façon usuelle en des lames flexibles, les organes de blocage des dits organes de verrouillage sont alors avantageusement adaptés, selon l'invention, pour interdire, dans leur position de désactivation, la déformation par flexion de ces derniers.

D'autres caractéristiques, buts et avantages de l'invention ressortiront de la description détaillée qui suit en référence aux dessins annexés qui en représentent à titre d'exemples non limitatifs deux modes de réalisation préférentiels. Sur ces dessins :
- la figure 1 a est une vue en perspective représentant un élément de verrouillage adapté pour équiper un premier mode de réalisation d'un dispositif d'injection selon l'invention représenté vu en perspective à la figure 1b,
- la figure 2 est une vue longitudinale d'une tige de piston d'un dispositif d'injection selon l'invention,
- les figures 3 et 4 sont des coupes longitudinales respectivement par un plan A et un plan axial B, du premier mode de réalisation de dispositif d'injection conforme à l'invention représenté en perspective aux figures 1 a et 1 b,
- la figure 5a est une vue en perspective représentant un élément de verrouillage adapté pour équiper un second mode de réalisation d'un dispositif d'injection selon l'invention représenté vu en perspective à la figure 5b,
- les figures 6 et 7 sont des coupes longitudinales respectivement par le plan axial B et le plan A, du second mode de réalisation de dispositif d'injection conforme à l'invention représenté en perspective aux figures 5a et 5b,
- les figures 8 et 9 sont deux coupes longitudinales partielles du premier mode de réalisation de dispositif d'injection conforme à l'invention représentant chacune, en demi coupe par le plan A et en demi coupe par le plan B, une des étapes du fonctionnement de ce dispositif d'injection,
- la figure 10 est une vue longitudinale du premier mode de réalisation de dispositif d'injection conforme à l'invention représentant ce dernier lors de l'étape finale de fin d'injection,
- et la figure 11 est une coupe longitudinale partielle du second mode de réalisation de dispositif d'injection conforme à l'invention représentant, en demi coupe par le plan A et en demi coupe par le plan B, l'étape du fonctionnement de ce dispositif d'injection correspondant à celle représentée à la figure 8 en référence au premier dispositif d'injection.

Les deux variantes de dispositifs d'injection selon l'inventton représentés à titre d'exemples aux figures sont du type à usage unique et consistent en des dispositifs d'injection particulièrement adaptés pour l'injection de médicaments requérant une reconstitution (solvant - produit lyophilisé) avant injection, et nécessitant, à cet effet :
- une première vidange destinée à injecter le solvant renfermé dans le dispositif d'injection dans un flacon contenant le produit lyophilisé,
- une aspiration du produit reconstitué en vue du remplissage du dispositif d'injection,
- et l'injection proprement dite du produit médicamenteux.

De plus, ces deux variantes de dispositifs d'injection intègrent des dispositifs de protection adaptés pour permettre de protéger leur aiguille d'injection après usage.

En premier lieu, chacun de ces dispositifs d'injection comprend un récipient tel qu'en l'exemple une seringue classique 1 constituée d'un tube de verre, d'une part, prolongé d'une embase 2 dans laquelle est scellée une aiguille d'injection 3, et d'autre part, doté d'une collerette externe 4 au niveau de son extrémité opposée à l'embase 2.

De plus, cette seringue 1 est classiquement équipée d'un piston 5 apte à coulisser de façon étanche à l'intérieur de cette dernière, et solidarisé, en vue de son actionnement, sur une extrémité d'une tige de piston 6 dotée, au niveau de son extrémité opposée, d'un bouton poussoir 7 présentant la forme d'une coupelle, c'est-à-dire présentant une face 7a de jonction avec la tige de piston 6, de forme convexe.

Tel que précité, ces deux dispositifs d'injection comprennent, en outre, un dispositif de protection après usage de l'aiguille d'injection 3. Selon les deux exemples représentés aux figures, ces deux dispositifs de protection consistent en des dispositifs de même conception de base que celle des dispositifs d'injection commercialisés par la société « SAFETY SYRINGES, Inc.», et décrits notamment dans les demandes de brevet WO 2006/127484 et WO 2006/050304, et les brevets US 6,030,366 et US 6,623,459, ces documents étant considérés, du fait de leur référencement, comme faisant partie intégrante de la présente description.

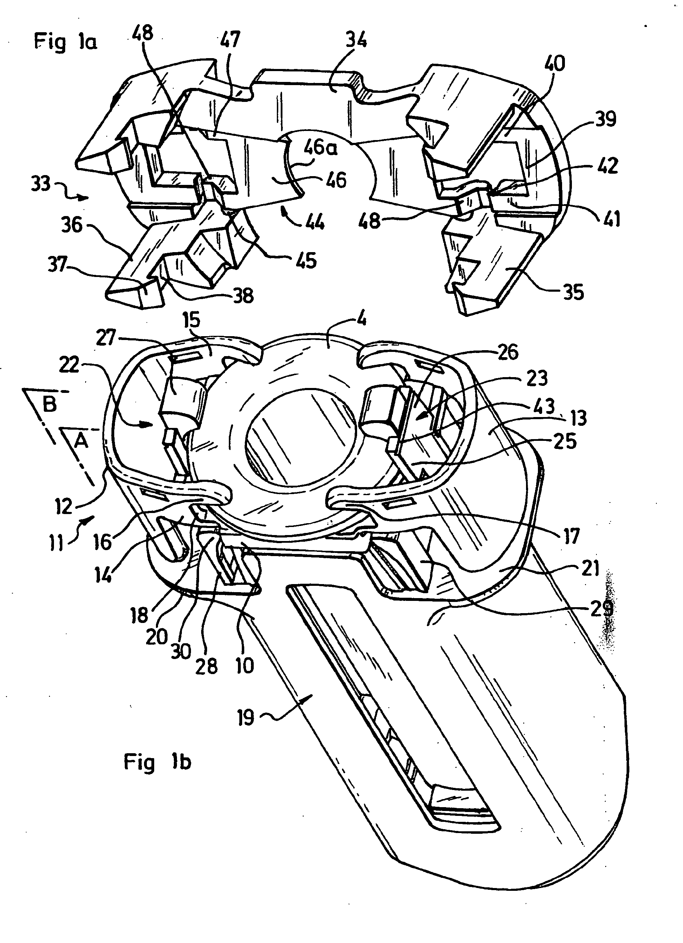

En premier lieu, le dispositif de protection du premier dispositif d'injection représenté aux figures 1b, 3 et 4, comporte un corps de seringue 8 du type décrit dans la demande de brevet WO 20061050304, comprenant un conduit longitudinal 9 définissant un logement pour la seringue 1, doté, au niveau d'une de ses extrémités, d'une couronne externe 10 de butée et d'appui de la collerette 4 de la dite seringue.

Ce corps de seringue 8 comporte, en outre, une tête tubulaire 11 de prise digitale, ménagée autour du tronçon d'extrémité du conduit longitudinal 9 sur lequel est formée la couronne 10.

Cette tête tubulaire 11 est formée de deux demi coquilles 12, 13 s'étendant symétriquement de part et d'autre de la couronne et reliées chacune à cette dernière par deux bras parallèles prolongés, chacun, d'une gâchette 16, 17 déformable élastiquement, et adaptés pour que les gâchettes 16, 17 de deux bras coplanaires définissent, avec la face en vis-à-vis de la couronne 10, une lumière 18 de logement et de blocage de la collerette externe 4 de la seringue 1.

Le dispositif de protection du premier dispositif d'injection conforme à l'invention comprend également un étui de protection 19 adapté pour coulisser extérieurement le long du conduit tubulaire 9 du corps de seringue 8.

Cet étui de protection 19 comporte, en premier lieu, deux ailettes de prise digitale 21, 22 disposées et de formes adaptées pour obturer sensiblement la face ouverte en regard de la tête tubulaire 11.

De plus, cet étui de protection 19 comporte deux doigts de verrouillage 22, 23 agencés pour s'étendre symétriquement de part et d'autre du conduit tubulaire 9, chacun à partir d'une des ailettes de prise digitale 20, 21, et adaptés pour bloquer en translation l'étui de protection 19 relativement au corps de seringue 8, dans une position relative rétractée du dit étui de protection où l'aiguille d'injection 3 dépasse de ce dernier.

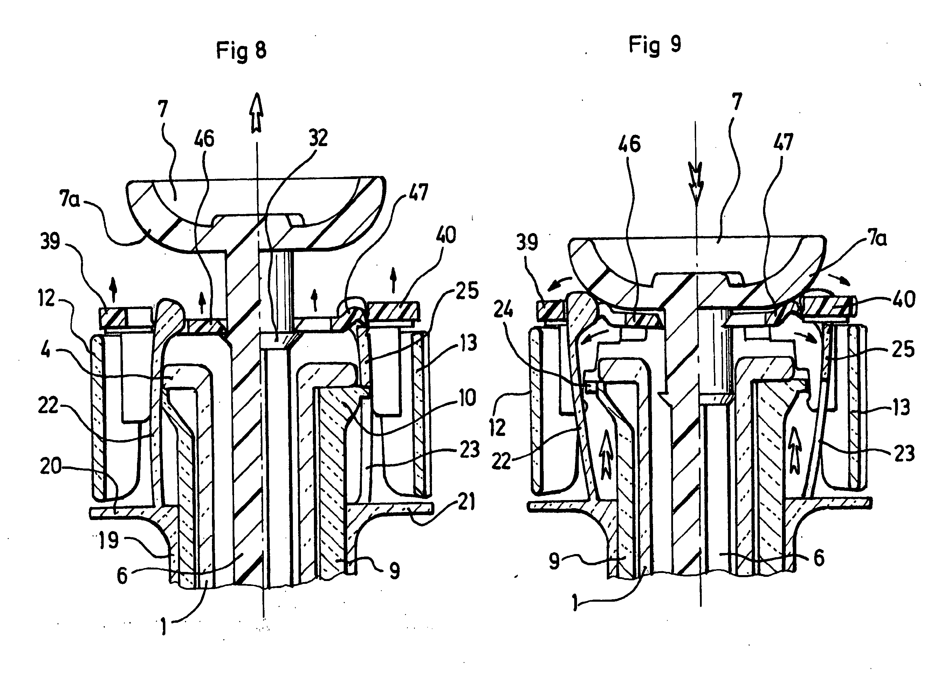

Chacun de ces doigts de verrouillage 22, 23 se compose d'une lame déformable élastiquement se prolongeant au-delà de la collerette 4 de la seringue 1 bloquée dans les lumières 18, et s'étendant notamment, à cet effet à l'intérieur d'une encoche 24, représentée à la figure 9, ménagée sur la périphérie de la couronne 10 du corps de seringue 8.

Chaque lame 22, 23 comporte, en outre, un organe intermédiaire de verrouillage adapté pour venir reposer et se verrouiller sur la couronne 10 de part et d'autre de l'encoche 24 ménagée dans cette dernière, le dit organe de verrouillage consistant en une traverse définissant deux plots de verrouillage 25, 26 s'étendant de part et d'autre de la dite lame.

Chaque lame 22, 23 comporte, enfin, un bourrelet 27 ménagé au niveau de son extrémité libre et adapté pour former une face d'appui complémentaire de la face convexe du bouton poussoir 7 de la tige de piston 6, apte a déclencher un déverrouillage des plots de verrouillage 25, 26 par déformation de chaque lame 22, 23 en fin de course de la dite tige de piston 6.

L'étui de protection comporte également quatre doigts de verrouillage tels que 28, 29 répartis autour du conduit tubulaire 9, agencés pour s'étendre chacun à partir d'une des ailettes de prise digitale 20, 21, et adaptés pour bloquer en translation l'étui de protection 19 relativement au corps de seringue 8, dans une position relative déployée du dit étui de protection où l'aiguille d'injection 3 est entièrement logée dans ce dernier.

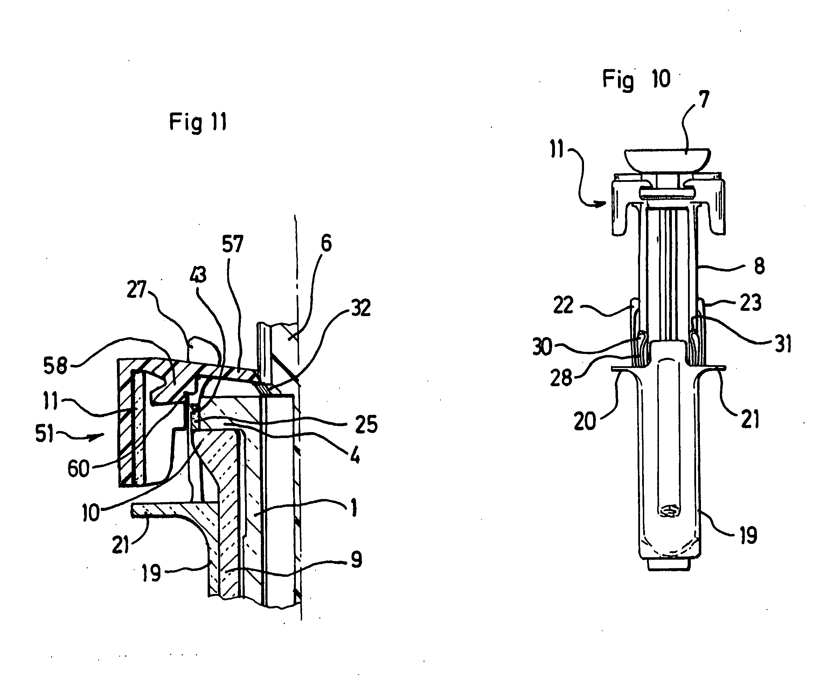

Chacun de ces doigts de verrouillage 28. 29 se compose d'une lame déformable élastiquement, dotée, au niveau de son extrémité libre, d'un ergot de verrouillage 30 adapté pour venir se loger dans un cran 31 ménagé le long du conduit longitudinal 9, et représenté à la figure 10.

De façon usuelle, le dispositif de protection comporte également un ressort (non représenté) disposé entre le corps de seringue 8 et l'étui de protection 19 et adapté pour entraîner un coulissement relatif des ces derniers en fin d'injection.

Selon l'invention et, en premier lieu, la tige de piston 6 comporte une collerette 32 ménagée en position intermédiaire de la longueur de la dite tige de piston.

Selon l'invention, en outre, les dispositifs de protection comportent un élément de verrouillage adapté pour collaborer avec la collerette 32 précitée en vue de présenter, de façon automatique, un état apte :
- à interdire le déclenchement du déverrouillage des plots de déverrouillage 25, 26, lors du premier actionnement de la tige de piston 6 visant à permettre le mélange du produit,
- et à autoriser le déclenchement du déverrouillage des plots de déverrouillage 25, 26, lors du second actionnement de la tige de piston 6 destiné à l'injection du produit.

Concernant le dispositif de protection du premier mode de réalisation de dispositif d'injection selon l'invention, cet élément de verrouillage 33 est adapté pour s'emboîter à l'intérieur de la tête de prise digitale 11.

Il comprend une plaque de verrouillage 34 adaptée pour s'étendre sensiblement dans le plan de la face de la tête de prise digitale opposée aux ailes de prise digitale 20, 21, par rapport à laquelle s'étendent quatre pieds tels que 35, 36 adaptés pour s'étendre à l'intérieur de la tête de prise digitale 11, et se terminant par un crochet 37 délimitant une gorge longitudinale 38 adaptée pour loger, avec un jeu de débattement, le rebord de la couronne 10.

La plaque de verrouillage 34 comprend, en outre, en regard de chaque doigt de verrouillage 22, 23, une découpe en forme de U 39 définissant deux branches parallèles 40, 41 adaptées pour se positionner en butée chacune contre un plot de verrouillage 25, 26.

En vue de parfaire le verrouillage des doigts de verrouillage 22, 23, la face d'extrémité de chaque branche 40, 41 présente, en outre, la forme d'un ergot 42 adapté pour se loger dans un cran 43 de forme complémentaire ménagé dans la face en regard de chaque plot de verrouillage 25,26.

Cette plaque de verrouillage 34 comprend, en outre, associé à chaque doigt de verrouillage 22, 23, un levier 44 articulé autour d'un axe de pivotement 45 orthogonal à l'axe longitudinal du corps de seringue 8, le dit levier comportant :
- s'étendant sur un des côtés de l'axe de pivotement 45, une pièce plane 46 de butée de la collerette intermédiaire 32 de la tige de piston 6, ménagée de façon à être située sur la trajectoire de la dite collerette, la dite pièce de butée présentant, en outre, un bord libre 46a biseauté apte à faciliter le passage de la collerette 32 lors d'un déplacement du piston 5 dans le sens de la vidange de la seringue 1,
- et s'étendant de l'autre côté de l'axe de pivotement 45, deux branches parallèles 47, 48 chacune adaptée pour venir en appui, au niveau de son extrémité libre, sur un plot de verrouillage 25, 26.

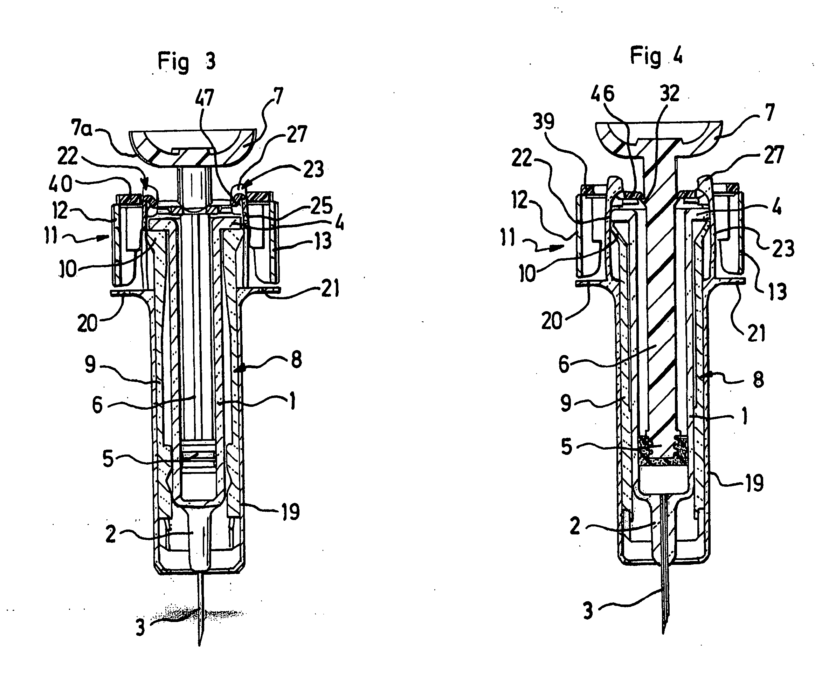

Le fonctionnement de ce premier dispositif d'injection est décrit ci-dessous en référence aux figures 3, 4, 8, 9 et 10.

Dans la position initiale du dispositif d'injection représentée aux figures 3 et 4, l'élément de verrouillage 33 est positionné de façon que les ergots 42 des branches 40, 41 soient logés dans les crans 43 des plots de verrouillage 25, 26.

Cet élément de verrouillage 33 se trouve ainsi dans une position dite de désactivation dans laquelle il interdit tout écartement relatif des doigts de verrouillage 22, 23, et par conséquent tout déverrouillage des dits doigts de verrouillage.

Le premier actionnement du piston 5 visant à vidanger la seringue en vue du mélange du solvant et du produit lyophilisé, n'engendre aucune modification de la position de l'élément de verrouillage 33 : le passage par la collerette intermédiaire 32 de la tige de piston 6 de l'obstacle que constitue la pièce de butée 46 n'engendre, en effet, qu'un basculement sans conséquence du levier 44.

Par contre, tel que représenté à la figure 8, lors de l'aspiration du mélange à l'intérieur de la seringue 1, la collerette intermédiaire 32 vient buter contre la pièce de butée 46 et entraîne un basculement du levier 44 qui provoque un arc-boutement des branches 47, 48 sur les plots de verrouillage 25, 26 : l'élément de verrouillage 33 subit un débattement axial et vient se positionner dans un état dit d'activation dans lequel les branches 40, 41 sont disposées au-delà des plots de verrouillage 25, 26 et ne s'opposent donc plus à l'écartement relatif des doigts de verrouillage 22, 23.

De ce fait, et tel que représenté à la figure 9, lorsque le piston 5 parvient en fin de course, le bouton poussoir 7 déclenche le déverrouillage des doigts de déverrouillage 22, 23, et entraîne ainsi un coulissement relatif du corps de seringue 8 et de l'étui de protection 19 jusqu'à obtention d'une position relative déployée de l'étui de protection 19, représentée à la figure 10, matérialisée par la coopération des ergots de verrouillage 30 et des crans 31, et dans laquelle l'aiguille d'injection 3 est logée à l'intérieur du dit étui de protection.

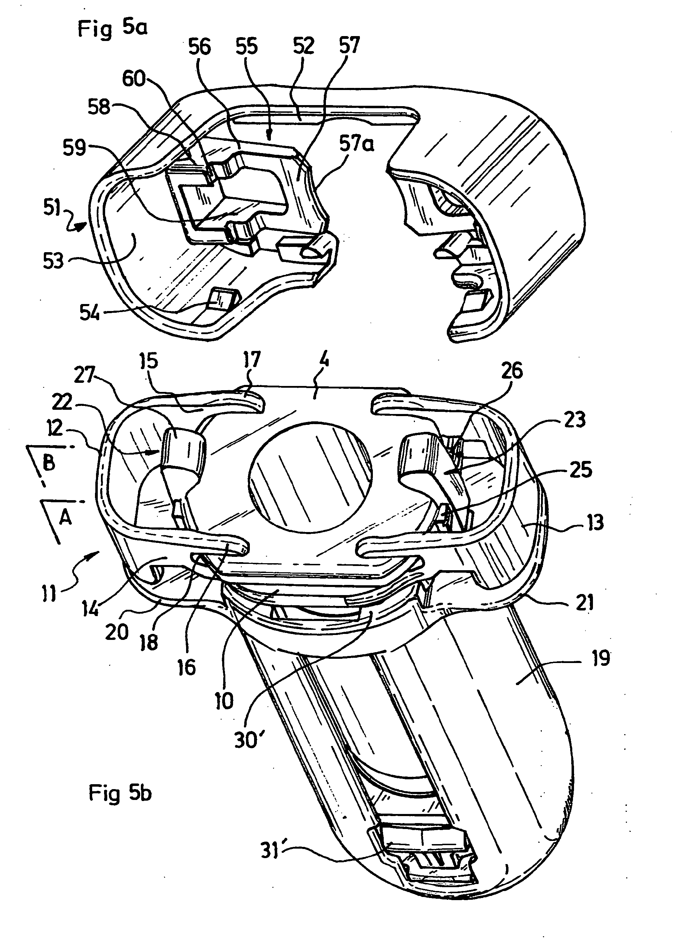

Le dispositif de protection du second mode de réalisation de dispositif d'injection conforme à l'invention représenté aux figures 5b, 6 et 7, comporte un corps de seringue 8 et un étui de protection similaires à ceux ci-dessus décrits.

Les seules différences marquantes sont relatives aux organes de verrouillage adaptés pour bloquer en translation l'étui de protection 19 relativement au corps de seringue 8 dans la position relative déployée du dit étui de protection où l'aiguille d'injection 3 est entièrement logée dans ce dernier.

Selon ce mode de réalisation, en effet ces organes de verrouillage comprennent :
- une lumière 30' ménagée dans la paroi périphérique de la tête de prise digitale 11,
- et un cran 31' formé sur le conduit longitudinal 9 du corps de seringue 8, vers l'extrémité libre de ce dernier.

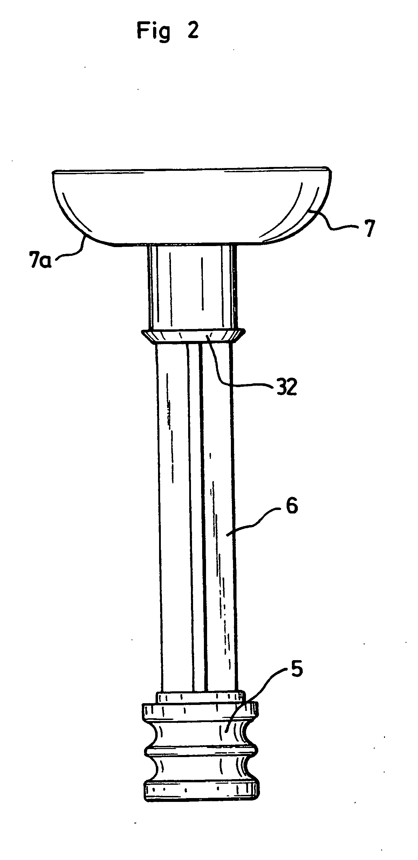

De même, la tige de piston 6 est similaire à celle ci-dessus décrite et représentée à la figure 2.

La différence essentielle entre ces deux modes de réalisation concerne l'élément de verrouillage.

Selon le second mode de réalisation, l'élément de verrouillage 51 est adapté pour coiffer la tête de prise digitale 11.

Il comprend une plaque de verrouillage 52 adaptée pour s'étendre sensiblement dans le plan de la face de la tête de prise digitale 11 opposée aux ailes de prise digitale 20, 21, par rapport à laquelle s'étend une jupe 53 adaptée pour coiffer les deux demi coquilles 12, 13.

De plus, cet élément de verrouillage 51 comporte des ergots tels que 54 ménagés en saillie par rapport à la face interne de la jupe 53 et adaptés pour s'encliqueter sans jeu sous les bras 14, 15 de la tête tubulaire 11.

Cet élément de verrouillage 51 comprend, en outre, associé à chaque doigt de verrouillage 22, 23, une pièce de verrouillage 55 disposée à l'intérieur de la jupe 53, la dite pièce de verrouillage présentant une élasticité propre et étant reliée à la jupe 53 par une liaison telles qu'elle présente une faculté de déformation élastique.

Cette pièce de verrouillage 55 comporte :
- un cadre 56 définissant une ouverture pour le passage d'un des doigts de verrouillage 22, 23, le dit cadre comportant, en outre, une traverse 57 de butée de la collerette intermédiaire 32 de la tige de piston 6, ménagée de façon à être située sur la trajectoire de la dite collerette, la dite traverse de butée présentant, en outre, un bord libre 57a biseauté apte à faciliter le passage de la collerette 32, lors d'un déplacement du piston 5 dans le sens de la vidange de la seringue 1,
- deux branches 58, 59 s'étendant chacune sous un des montants du cadre 56 perpendiculaire à la traverse 57, de façon à se positionner en butée chacune contre un plot de verrouillage 25, 26 des doigts de verrouillage 22, 23, les dites branches présentant une face d'extrémité formant un ergot 60 adapté pour se loger dans le cran 43 du plot de verrouillage 25, 26 en vis à vis.

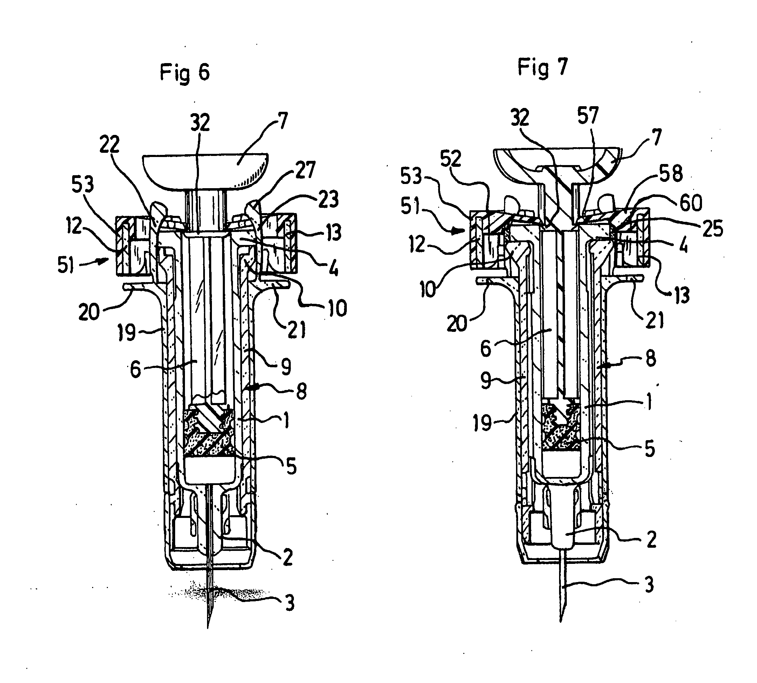

Selon ce principe l'élément de verrouillage 51 est fixé sans jeu sur la tête de prise digitale 11, et se trouve initialement positionné, tel que représenté aux figures 6 et 7, de façon que les ergots 60 des branches 58, 59 soient logés dans les crans 43 des plots de verrouillage 25, 26.

Cet élément de verrouillage 51 se trouve ainsi dans une position de désactivation dans laquelle il interdit tout écartement relatif des doigts de verrouillage 22, 23, et par conséquent tout déverrouillage des dits doigts de verrouillage.

Tel que précédemment, le premier actionnement du piston 5 visant à vidanger la seringue n'engendre aucune modification de la position de l'élément de verrouillage.

Par contre, tel que représenté à la figure 11, lors de l'aspiration du mélange à l'intérieur de la seringue 1, la collerette intermédiaire 32 vient buter contre la traverse 57 et entraîne une déformation de la pièce de verrouillage 55 et notamment des branches 58, 59 qui entraînent un positionnement des ergots 60 au-delà des plots de verrouillage 25, 26, pour lequel les dites branches ne s'opposent plus à un écartement relatif des doigts de verrouillage 22, 23.

Moyennant l'adjonction d'un simple élément de verrouillage, l'invention conduit donc à transformer un dispositif d'injection sécurisé à usage unique en un dispositif d'injection sécurisé permettant, sans nécessiter une quelconque action spécifique de l'utilisateur, un premier actionnement du piston, puis l'injection proprement dite accompagnée de la sécurisation.

## Revendications

1. Dispositif sécurisé d'injection d'un produit médicamenteux tel qu'un produit lyophilisé, comprenant un corps de seringue (8) comportant un récipient (1) portant une aiguille d'injection (3) et doté d'un piston (5) actionné par une tige de piston (6) dotée d'un bouton poussoir (7), et un dispositif de protection comportant :
• un étui de protection (19) adapté pour que le dit étui de protection et le corps de seringue (8) soient aptes à coulisser relativement l'un par rapport à l'autre entre une position d'injection dans laquelle l'aiguille d'injection (3) s'étend au moins partiellement dans le prolongement de l'étui de protection (19), et une position de protection après usage dans laquelle l'aiguille d'injection (3) est entièrement logée dans l'étui de protection (19),
• au moins un organe de verrouillage (22, 23) apte à présenter un état verrouillé de maintien du corps de seringue (8) et de l'étui de protection (19) dans leur position d'injection, et pour commuter vers un état déverrouillé apte à autoriser, en fin de course du piston (5) à l'intérieur du récipient (1), le coulissement relatif du corps de seringue (8) et de l'étui de protection (19) vers leur position de protection après usage,
• un épaulement (32) ménagé en position intermédiaire de la longueur de la tige de piston (6),
• et un élément de verrouillage (33 ; 51) solidarisé sur l'un des éléments, corps de seringue (8) ou étui de protection (19), le dit dispositif d'injection étant **caractérisé en ce que** l'élément de verrouillage comporte :
• des organes (40, 41 ; 58 ; 59) de blocage, des organes de verrouillage,(22, 23) aptes à être déplacés entre une position initiale naturelle, dite de désactivation, de blocage de chaque organe de verrouillage (22, 23) dans son état verrouillé, et une position, dite d'activation, d'autorisation d'une commutation de chaque organe de verrouillage (22, 23) vers son état déverrouillé,
• et des organes d'actionnement (44 ; 57) reliés aux organes de blocage (40, 41 ; 58 ; 59), positionnés sur le trajet de l'épaulement intermédiaire (32) de la tige de piston (6), et adaptés pour subir un changement d'état entraînant un déplacement des dits organes de blocage de leur position de désactivation vers leur position d'activation, lors d'une sollicitation exercée sur les dits organes d'actionnement par l'épaulement intermédiaire (32), lors d'un déplacement de la tige de piston (6) résultant d'une traction exercée sur le bouton poussoir (7).

2. Dispositif d'injection selon la revendication 1 **caractérisé en ce que** :
• l'élément de verrouillage (33) est solidarisé sur un des éléments, corps de seringue (8) ou étui de protection (19), avec une faculté de débattement selon une direction parallèle à l'axe longitudinal du dispositif de protection, entre deux positions définissant les positions respectives de désactivation et d'activation des organes de blocage (40, 41) des organes de verrouillage (22, 23),
• les organes d'actionnement comportent au moins un levier (44) articulé sur l'élément de verrouillage (33) autour d'un axe de pivotement (45) et comportant, s'étendant de part et d'autre du dit axe de pivotement :
• une zone de contact (46) située sur le trajet de l'épaulement intermédiaire (32) de la tige de piston (6),
• et au moins une pièce d'arc-boutement (47, 48) apte à engendrer un débattement de l'élément de verrouillage (33) entraînant un déplacement des organes de blocage (40, 41) de leur position de désactivation vers leur position d'activation, lors d'un pivotement du levier (44) provoqué par le déplacement de la tige de piston (6) résultant d'une traction exercée sue le bouton poussoir (7).

3. Dispositif d'injection selon la revendication 1 **caractérisé en ce que** :
• l'élément de verrouillage (51) est solidarisé en position fixe sur un des éléments, corps de seringue (8) ou étui de protection (19),
• les organes d'actionnement comportent au moins une pièce de verrouillage (55) solidaire de l'élément de verrouillage (51), comportant d'un seul tenant
• une zone de contact (57) située sur le trajet de l'épaulement intermédiaire (32) de la tige de piston (6),
• au moins un organe (58, 59) de blocage d'un organe de verrouillage (22, 23),
• et une zone déformable (56) de liaison entre la zone de contact (57)et chaque organe de blocage (58 ; 59), apte à se déformer et à entraîner un déplacement de chaque organe de blocage (58 ; 59) de sa position de désactivation vers sa position d'activation, lors d'une sollicitation exercée sur la zone de contact (57) par l'épaulement intermédiaire (32), lors d'un déplacement de la tige de piston (6) résultant d'une traction exercée sue le bouton poussoir (7).

4. Dispositif d'injection selon l'une des revendications précédentes **caractérisé en ce que** l'élément de verrouillage (33 ; 51) est solidarisé sur le corps de seringue (8).

5. Dispositif d'injection selon l'une des revendications précédentes **caractérisé en ce que** les organes de blocage (40, 41 ; 58 ; 59) de l'élément de verrouillage (33 ; 51) et les organes de verrouillage (22, 23) de maintien du corps de seringue (8) et de l'étui de protection (19) dans leur position d'injection, comprennent des organes d'assemblage amovible (42, 43 ; 60, 43) complémentaires, du type cran/ ergot.

6. Dispositif d'injection selon l'une des revendications précédentes dont les organes de verrouillage (22, 23), de maintien du corps de seringue (8) et de l'étui de protection (19) dans leur position d'injection, consistent en des lames flexibles, **caractérisé en ce que** les organes de blocage (40, 41 ; 58 ; 59) des dits organes de verrouillage, sont adaptés pour interdire, dans leur position de désactivation, la déformation par flexion de ces derniers.

## Patentansprüche

1. Geschützte Vorrichtung zur Injektion eines medikamentösen Produktes, wie ein lyophilisiertes Produkt, mit einem Spritzenkörper (8) mit einem Behälter (1), der eine Injektionsnadel (3) trägt und mit einem Kolben (5) versehen ist, der durch einen Kolbenstift (6) betätigt wird, der mit einem Druckknopf (7) versehen ist, und eine Schutzvorrichtung mit:
• einer Schutzhülle (19), die angepasst ist, damit die Schutzhülle und der Spritzenkörper (8) geeignet sind, relativ in Bezug zueinander zwischen eine Injektionsposition zu gleiten, in welcher sich die Injektionsnadel (3) zumindest teilweise in die Verlängerung der Schutzhülle (19) erstreckt, und einer Schutzposition nach Verwendung in welcher die Injektionsnadel (3) vollständig in der Schutzhülle (19) untergebracht ist,
• zumindest einem Verriegelungsorgan (22, 23), das geeignet ist, einen Verriegelungszustand zum Halten des Spritzenkörpers (8) und der Schutzhülle (19) in ihrer Injektionsposition bereitzustellen, und zum Schalten in einen entriegelten Zustand, der geeignet ist am Ende des Hubes des Kolbens (5) an der Innenseite des Behälters (1) das relative Gleiten des Spritzenkörpers (8) und der Schutzhülle (19) in ihre Schutzposition nach Verwendung zuzulassen,
• einem Anschlag (32), der in mittlerer Position der Länge des Kolbenstiftes (6) vorgesehen ist,
• und einem Verriegelungselement (33; 51), das auf einem der Elemente, Spritzenkörper (8) oder Schutzhülle (19), verbunden ist,
wobei die Injektionsvorrichtung **dadurch gekennzeichnet ist, dass** das Verriegelungselement aufweist:
• Blockierorgane (40; 41; 58; 59), Verriegelungsorgane (22, 23), die geeignet sind zwischen eine native Anfangsposition versetzt zu werden, sozusagen zur Deaktivierung, zur Blockade von jedem Verriegelungsorgan (22, 23) in dessen Verriegelungszustand und einer Position, sozusagen zur Aktivierung, zum Zulassen einer Schaltung von jedem Verriegelungsorgan (22, 23) in dessen entriegeltem Zustand,
• und Betätigungsorgane (44; 57), die mit den Blockierorganen (40, 41; 58; 59) verbunden sind, die auf dem Weg des mittleren Anschlags (32) des Kolbenstiftes (6) angeordnet sind und angepasst sind, um eine Zustandsänderung zu erfahren, die eine Versetzung der Blockierorgane von ihrer Deaktivierungsposition in ihre Aktivierungsposition herbeiführt, bei einer ausgeübten Belastung auf die Betätigungsorgane durch den mittleren Anschlag (32) bei einer Versetzung des Kolbenstiftes (6), die durch einen auf dem Druckknopf (7) ausgeübten Zug eingeführt wird.

2. Injektionsvorrichtung nach Anspruch 1,
**dadurch gekennzeichnet, dass**
• das Verriegelungselement (33) auf einem der Elemente, Spritzenkörper (8) oder Schutzhülle (19), verbunden ist, mit einer Befähigung in eine parallele Richtung der Längsachse der Schutzvorrichtung zwischen zwei Positionen, die die jeweiligen Positionen der Deaktivierung und der Aktivierung der Blockierorgane (40, 41) der Verriegelungsorgane (22, 23) definieren, auszulen-ken,
• die Betätigungsorgange zumindest einen Hebel (44) aufweisen, der gelenkig auf dem Verriegelungselement (33) um eine Schwenkachse (45) herum angebunden ist, und der auf beiden Seiten der Schwenkachse erstreckend aufweist:
• einen Kontaktbereich (46), der auf dem Weg des mittleren Anschlags (32) des Kolbenstifts (6) angeordnet ist,
• und zumindest ein Verklemmungsteil (47, 48), das geeignet ist, eine Auslenkung des Verriegelungselementes (33) zu erzeugen, das eine Versetzung der Blockierorgane (40, 41) von ihrer Deaktivierungsposition in ihre Aktivierungsposition herbeiführt, bei einer Neigung des Hebels (44), die durch die Versetzung des Kolbenstiftes (6) verursacht wird, die durch einen auf dem Druckknopf (7) ausgeübten Zug engeführt wird.

3. Injektionsvorrichtung nach Anspruch 1,
**dadurch gekennzeichnet, dass**
• das Verriegelungselement (51) in feststehender Position auf einem der Elemente, Spritzenkörper (8) oder Schutzhülle (19), verbunden ist,
• die Betätigungsorgane zumindest ein Verriegelungsteil (55) aufweisen, das mit dem Verriegelungselement (51) fest verbunden ist, mit in einem Stück
• einem Kontaktbereich (57), der auf dem Weg des mittleren Anschlags (32) des Kolbenstiftes (6) angeordnet ist,
• zumindest einem Blockierorgan (58, 59) des Verriegelungsorgans (22, 23),
• und einem verformbaren Verbindungsbereich (56) zwischen dem Kontaktbereich (57) und jedem Blockierorgan (58; 59), der geeignet ist sich zu verformen und eine Versetzung von jedem Blockierorgan (58; 59) von dessen Deaktivierungsposition in dessen Aktivierungsposition herbeizuführen bei einer ausgeübten Belastung auf den Kontaktbereich (57) durch den mittleren Anschlag (32) bei einer Versetzung des Kolbenstiftes (6), die durch einen auf dem Druckknopf (7) ausgeübten Zug eingeführt wird.

4. Injektionsvorrichtung nach einem der vorstehenden Ansprüche,
**dadurch gekennzeichnet, dass**
das Verriegelungselement (33; 51) auf dem Spritzenkörper (8) verbunden ist.

5. Injektionsvorrichtung nach einem der vorstehenden Ansprüche,
**dadurch gekennzeichnet, dass**
die Blockierorgane (40, 41; 58; 59) des Verriegelungselementes (33; 51) und die Verriegelungsorgane (22, 23) zum Halten des Spritzenkörpers (8) und der Schutzhülle (19) in deren Injektionsposition abnehmbare komplementäre Verbindungsorgane (42, 43; 60, 43) von der Art einer Stufe/Zapfen aufweisen.

6. Injektionsvorrichtung nach einem der vorstehenden Ansprüche,
wovon die Verriegelungsorgane (22, 23) zum Halten des Spritzenkörpers (8) und der Schutzhülle (19) in ihrer Injektionsposition aus flexiblen Klingen bestehen,
**dadurch gekennzeichnet, dass**
die Blockierorgane (40, 41; 58; 59) der Verriegelungsorgane angepasst sind, um in ihrer Deaktivierungsposition die Verformung durch Biegen dieser letzteren zu unterbinden.

## Claims

1. Safety device for injecting a medicinal product such as a lyophilized product, comprising a syringe body (8) with a receptacle (1) which has an injection needle (3) and equipped with a plunger (5) actuated by a plunger rod (6) equipped with a push-button (7), and a protection device comprising:
- a protective casing (19) adapted so that said protective casing and the syringe body (8) are able to slide relatively one in relation to the other between an injection position in which the injection needle (3) extends at least partially into the extension of the protective casing (19), and a protection position after use in which the injection needle (3) is completely accommodated in the protective casing (19),
- at least one locking member (22, 23) which can have a locked state for holding the syringe body (8) and the protective casing (19) in their injection position, and for switching to an unlocked state which can allow, at the end of the movement of the plunger (5) inside the receptacle (1), the relative sliding of the syringe body (8) and the protective casing (19) to their protection position after use,
- a shoulder (32) placed in an intermediate position of the length of the plunger rod (6), and
- a locking member (33; 51) attached on one of the members, syringe body (8) or protective casing (19),
said injection device being **characterised in that** the locking member comprises:
- blocking devices (40, 41; 58; 59) locking members (22, 23) that can be displaced between a natural initial position, called the deactivation position, of blocking each locking member (22, 23) in its locked state, and a position, called the activation position, which allows each locking member (22, 23) to be switched to its unlocked state,
- and actuating members (44; 57) connected to the blocking devices (40, 41; 58; 59), positioned on the trajectory of the intermediate shoulder (32) of the plunger rod (6), and adapted to undergo a change of state bringing about a displacement of said blocking devices from their deactivation position to their activation position, when a force is exerted on said actuating members by the intermediate shoulder (32), during a displacement of the plunger rod (6) resulting from a tension exerted on the push-button (7).

2. Injection device according to claim 1, **characterised in that**:
- the locking member (33) is attached on one of the members, syringe body (8) or protective casing (19), with an ability of movement according to a direction parallel to the longitudinal axis of the protection device, between two positions defining the respective positions of deactivation and activation of the blocking devices (40, 41) of the locking members (22, 23),
- the actuating members comprise at least one lever (44) articulated on the locking member (33) around a rotation axis (45) and comprising, extending on both sides of said rotation axis:
- a contact area (46) situated on the trajectory of the intermediate shoulder (32) of the plunger rod (6),
- and at least one buttressing part (47, 48) which can bring about a movement of the locking member (33) resulting in a displacement of the blocking devices (40, 41) from their deactivation position to their activation position, during a rotation of the lever (44) caused by the displacement of the plunger rod (6) resulting from a tension exerted on the push-button (7).

3. Injection device according to claim 1, **characterised in that**:
- the locking member (51) is attached in a fixed position on one of the members, syringe body (8) or protective casing (19),
- the actuating members comprise at least one locking part (55) integral with the locking member (51), comprising in a single piece
- a contact area (57) situated on the trajectory of the intermediate shoulder (32) of the plunger rod (6),
- at least one blocking device (58, 59) of one locking member (22, 23),
- and a deformable area (56) of connection between the contact area (57) and each blocking device (58; 59) which can deform and bring about a displacement of each blocking device (58; 59) from its deactivation position to its activation position, when a force is exerted on the contact area (57) by the intermediate shoulder (32) during a displacement of the plunger rod (6) resulting from a tension exerted on the push-button (7).

4. Injection device according to any one of the preceding claims, **characterised in that** the locking member (33; 51) is attached on the syringe body (8).

5. Injection device according to any one of the preceding claims, **characterised in that** the blocking devices (40, 41; 58; 59) of the locking member (33; 51) and the locking members (22, 23) for holding the syringe body (8) and the protective casing (19) in their injection position, comprise additional removable assembly members (42, 43; 60, 43) of the catch/pin type.

6. Injection device according to any one of the preceding claims, in which the locking members (22, 23), for holding the syringe body (8) and the protective casing (19) in their injection position, consist of flexible strips, **characterised in that** the blocking devices (40, 41; 58; 59) of said locking members, are adapted to prevent, in their deactivation position, the deformation of the latter by bending.
